# EUROPEAN PATENT APPLICATION

(11) **EP 4 810 949 A1**
(43) Date of publication of application: **23.09.2026**
(21) Application number: 26162140.3
(22) Date of filing: 04.03.2026
(51) Int. Cl.: G01N 27/07, G01D 11/24, G01N 27/08, G01N 33/18

(54) **CONDUCTIVITY SENSOR ASSEMBLY**

(30) Priority: 18.03.2025 US 202519083059
(71) Applicant: Georg Fischer Signet LLC, Irwindale, CA 91706 (US)
(72) Inventor: Robertson, David Eugene, Glendora, CA 91741 (US); Bae, Youngsam, Placentia CA, 92870 (US)
(74) Representative: Li Schrag, Yue

(57) **Abstract**

A conductivity sensor including an elongated cylindrical body having major axis with an upper section and a lower section spaced from the upper section, the body having a bore extending along the major axis and through a through hole in the lower section. A drive coil and a receiving coil in the lower section. A plurality of pillars connecting a top of the lower section with a bottom of the upper section of the body while leaving radially extending gaps therebetween to allow fluid to flow through the gaps and then pass adjacent to the drive coil and receiving coil and through the through hole in the lower section.

## Description

### Field of the invention

The present disclosure relates to devices for measuring the conductivity of liquids and, more particularly, to conductivity sensors for sensing the conductivity of water quality.

### Background of invention

This section provides background information related to the present disclosure which is not necessarily prior art.

Contact conductivity sensors are ideally suited for measuring resistivity/conductivity of liquids ranging from pure and ultrapure water to sea water, rinse water and chemical solutions. Conductivity sensors generally rely on an electrodes that are carefully sized and positioned to enhance conductivity sensor accuracy. Some conductivity sensor embodiments utilize internally positioned electrodes to achieve the necessary sensor performance, which can contribute to electrode fouling that can severely affect electrode performance and, therefore, conductivity sensor accuracy. Because of these concerns, it is important that the conductivity sensor be easily cleaned which, because of the design of conventional conductivity sensors, typically requires removal of sensor from the sensing environment for cleaning and active scrubbing and internal electrodes are difficult to access and can be easily damaged. Instead, conventional conductivity sensors require frequent manual maintenance to ensure the sensor is clean and reading accurately.

Accordingly, there is a need in the art for a conductivity sensor that can be easily cleaned.

In addition, many conductivity sensors include a temperature sensor to sense the temperature of the fluid being measured. The temperature from the sensor is used to help measure the conductivity of the liquid, along with information supplied by the coils. It is, therefore, important that the information from the temperature sensor be consistently accurate.

### Summary of the invention

This section provides a general summary of the disclosure, and is not a comprehensive disclosure of its full scope or all of its features.

In accordance with the teachings of the present invention a conductivity sensor is provided having an elongated non-conductive body. First and second electrodes extend from the body and are vertically separated from each other.

Various embodiments of the invention are illustrated and described all which cooperate to provide a more robust conductivity sensor which lends itself to be easily cleaned and provides accurate measurements.

Further areas of applicability will become apparent from the description provided herein. The description and specific examples in this summary are intended for purposes of illustration only and are not intended to limit the scope of the present disclosure.

### Brief Description of the Drawings

The drawings described herein are for illustrative purposes only of selected embodiments and not all possible implementations, and are not intended to limit the scope of the present disclosure.

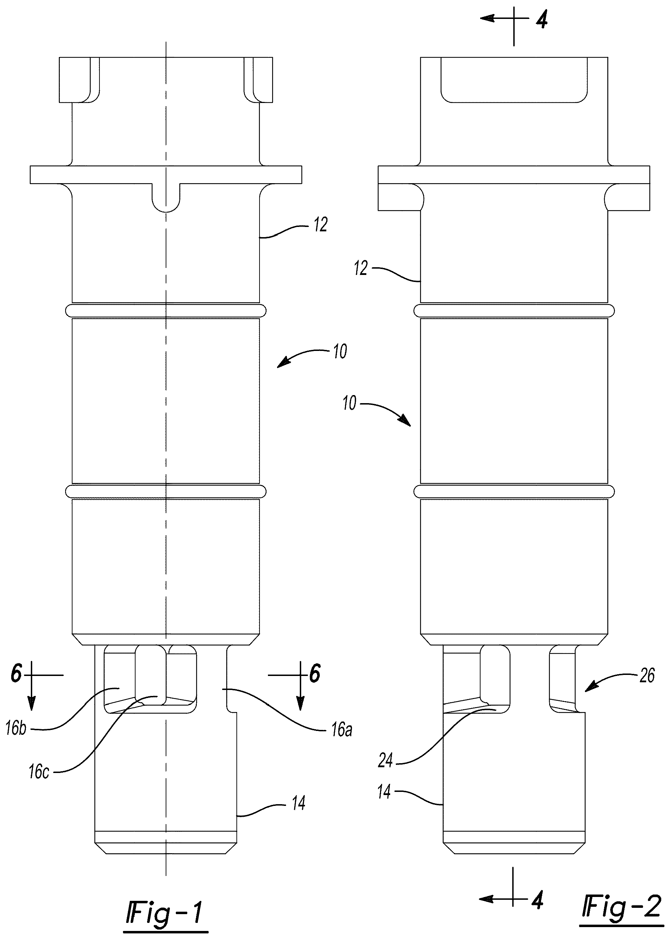

Fig. 1: illustrates a front view of a conductivity sensor made in accordance with the teachings of the present invention;
Fig. 2: illustrates a right side view of the conductivity sensor shown in Fig. 1;
Fig. 3: illustrates a left side view of the conductivity sensor shown in Fig. 1;
Fig. 4: illustrates a perspective view with parts cutaway of the conductivity sensor shown in Fig. 1;
Fig. 5: illustrates a cross-sectional view taken along the lines 5 - 5 of Fig. 3;
Fig. 6: illustrates a cross-sectional view taken along lines 6 - 6 of Fig. 1;
Fig. 6A: illustrates an enlarged cross-sectional view of the bottom portion of the conductivity sensor shown in Fig. 4;
Fig. 7: illustrates a front view of an alternative embodiment of conductivity sensor made in accordance with the teachings of this invention;
Fig. 8: illustrates a cross-sectional view along the lines 8 - 8 of Fig. 7;
Fig. 9: illustrates a side view of the conductivity sensor shown in Fig. 7;
Fig. 10: illustrates a side view of a secondary sensor;
Fig. 11: illustrates a cross-sectional view of a flushing nozzle;
Fig. 12: illustrates a side view of a bristle brush;
Fig. 13: illustrates the secondary sensor of Fig. 10 inserted into the sensor of Fig. 8;
Fig. 14: illustrates the flushing nozzle of Fig. 11 inserted into the sensor of Fig. 8; and
Fig. 15: illustrates the brush of Fig. 12 cleaning the through hole at the bottom of the sensor of Fig. 8.

### Exemplary embodiments

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular, order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Turning now to the drawings there is illustrated a conductivity sensor 10 made in accordance with the teachings of this invention. Sensor 10 has an elongated non-conductive tubular body of preferably made of chlorinated polyvinyl chloride (CVPC) pipe having an upper tubular section 12 which is connected to a spaced lower tubular section 14 by a plurality of pillars 16a, 16b and 16c. The lower section 14 has a centrally located through hole 18. Surrounding through hole 18 is a toroidal drive coil 20 stacked above a receiving toroidal coil 22. As is known in the art, electrical signals from drive coil 20 and toroidal coil 22 are used to sense characteristics of the liquid to be measured when the sensor 10 is immersed into the liquid.

One of the features of the present invention is to provide measures to keep the area between coil 20 and 22 clean so that they can provide accurate measurements of the liquid, for example, its conductivity. To this end, a radially inwardly directed upwardly sloping shoulder 24 is provided on the upper portion of lower section 14. This shoulder 24 is used to prevent debris from the liquid from settling into the through hole 18 when the pipeline flow of the liquid is turned off. The sloping surfaces will urge the debris away from the through hole 18, such debris otherwise being capable of disturbing the accuracy of the measurement.

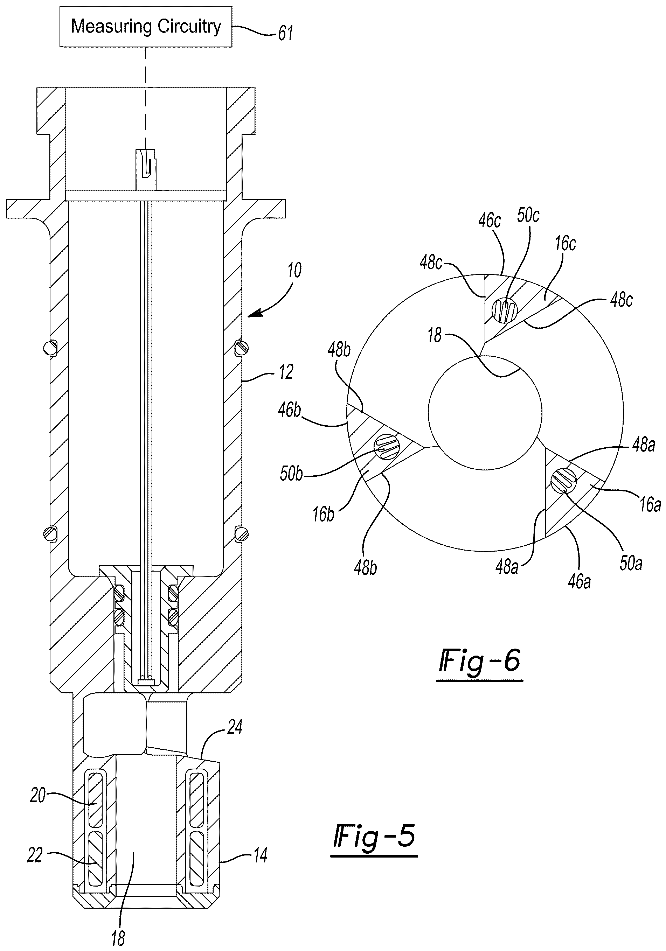

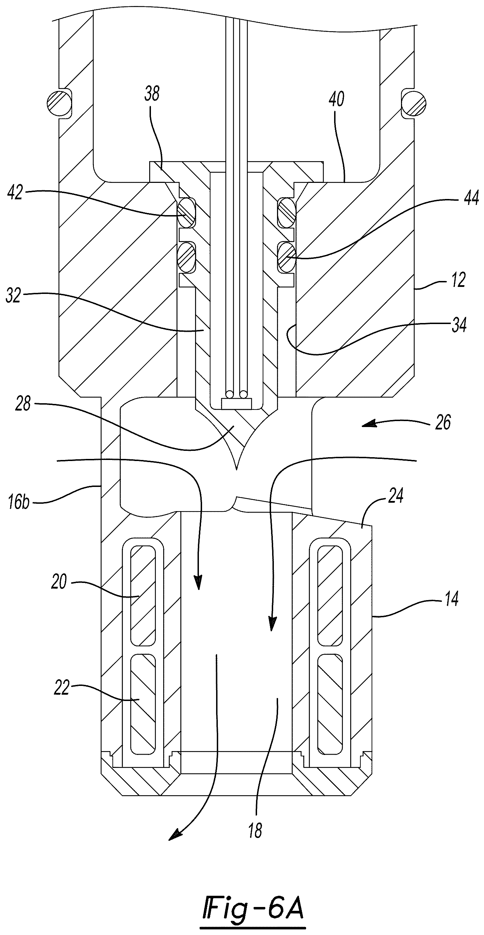

The spacing between pillars 16a - 16c provide gaps (generally designated by the numeral 26) between upper section 12 and the lower section 14. Thus, liquid to be measured can flow through the gaps 26 and into the through hole 18 and out of the bottom of the lower section 14. With particular reference to Fig. 6A a downwardly extending conical flow diverter 28 is provided to guide the liquid to be measured to the through hole 18. The flow diverter 28 serves to redirect water that would normally pass through gap 26. Flow diverter 28 directs the liquid through the through hole 18 improving conductivity response time and improving cleaning. If the liquid is stagnant in the through hole 18 it will slow conductivity response. The flow diverter 28 may also act to create turbulence in the liquid to help clean the passage through the through hole 18.

Conveniently, the flow diverter 28 may be part of a thermowell 30. Thermowell 30 has a generally U-shaped conductive body 32 located in the lower portion of section 12 above gaps 26. The thermowell 30 as annular sidewalls 32 connected by a lower bridge member 34. The thermowell sidewalls 32 are thermally isolated by an annular space 34 between most of the sidewalls 32 and the body of section 12. An RTD temperature sensor 36 is a bonded to the upper portion of the thermowell bridge member 34. An upper portion of the thermowell 34 has a flange 38 which rests on a shelf 40 in the body of the upper section 12. The thermowell 30 is fit into an opening in the body of the lower section 12. O rings 42, 44 serve to further isolate the temperature sensor 36. The cavity within the body of the thermowell 30 is preferably filled with thermal paste.

The thus described thermowell 30 configuration improves the temperature response time. The response time is reduced by limiting the thermal mass adjacent to the temperature sensor 36. The mass of the body of section 12 may slow the temperature response time, while the thermal paste in the body speeds thermal transfer to sensor 36. A sensor with a small thermal mass will respond faster than one with a large thermal mass.

Referring now especially to Fig. 6 which shows a cross-section of the pillars 16a, 16b and 16c, they are in the shape of radially inwardly extending fins. The outer portion of the fins have arcuate shaped segments 46a, 46b and 46c aligning with the outer surface of the lower section 14. From the edges of these segments, radially inwardly extending sides 48a, 48b, and 48c converge to a point adjacent the through hole 18. Opposite sides differ in length to provide each pillar with an asymmetrical shape. In such manner, the pillars essentially form a fan which create rotational turbulence or vortex flow of the liquid into the through hole 18 to promote self-cleaning. This construction creates an unbalanced pressure difference between upper and lower portions of the through hole 18. This will promote flow through the through hole 18 and prevent dead flow zones that could affect accurate conductivity measurement. The turbulent flow around the sensing coils 20, 22 promotes better heat transfer than would laminar flow, resulting in improved temperature response time.

The pillars also include vertically extending holes to provide conduits for wires 50a, 50b, and 50c that electrically connect the coils, 20, 22 to external measuring circuitry 61 for measuring characteristics of the liquid as a function of electrical signals generated by the coils, 20, 22. This construction provides a compact and robust design for the conductivity sensor 10.

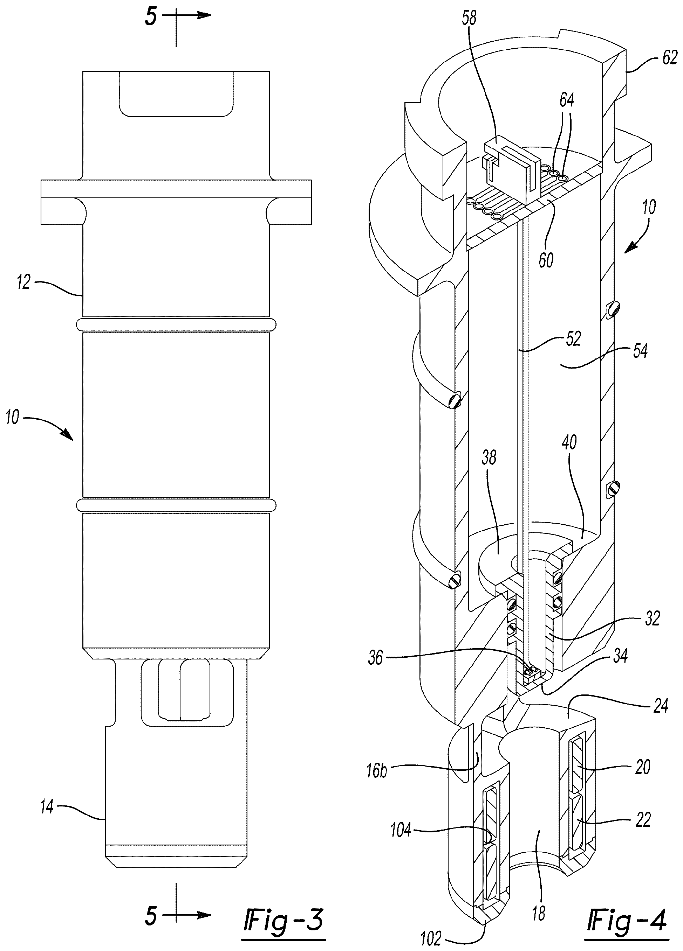

Referring more particularly to Fig. 4, sensor wires 52 pass through the main cavity 54 of the upper sensor section 12 from temperature sensor 36 to a ribbon connector 58 located on an upper shelf of a wire terminating board 60. The board 60 includes terminal pads 64 for terminating the RTD temperature sensor 36 and sensor wires 50a-50c. Cable connector 58 provides a convenient connection to an external cable connected to remotely located measurement electrical circuitry 61 for measuring the characteristics of the liquid, as well as the temperature of the liquid. The cavity 54 is preferably filled with potting compound. Bayonet lugs 62 are also provided for mounting sensor electronics.

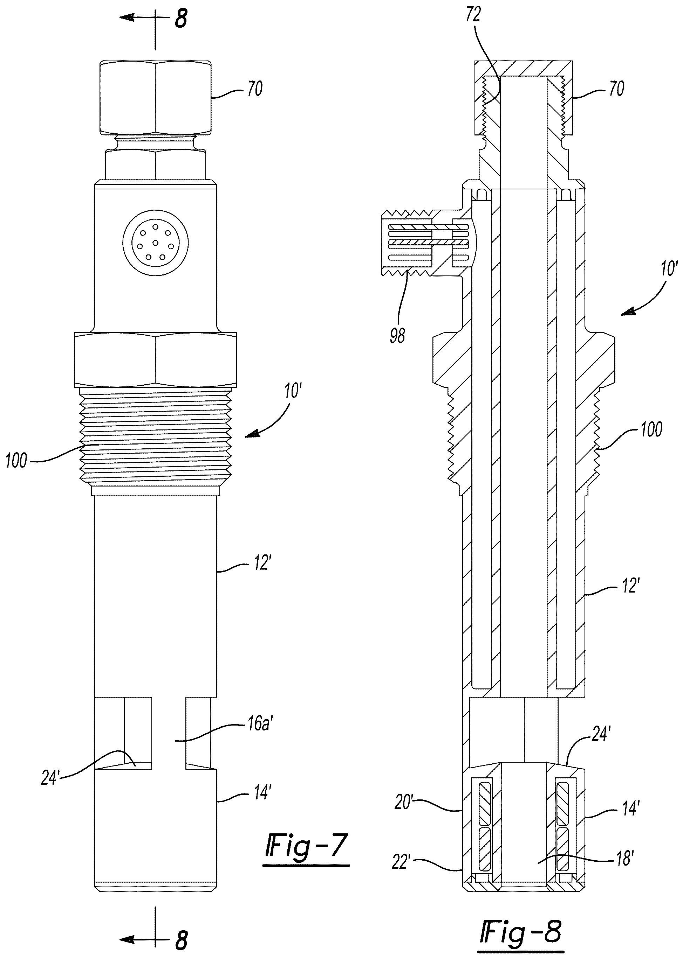

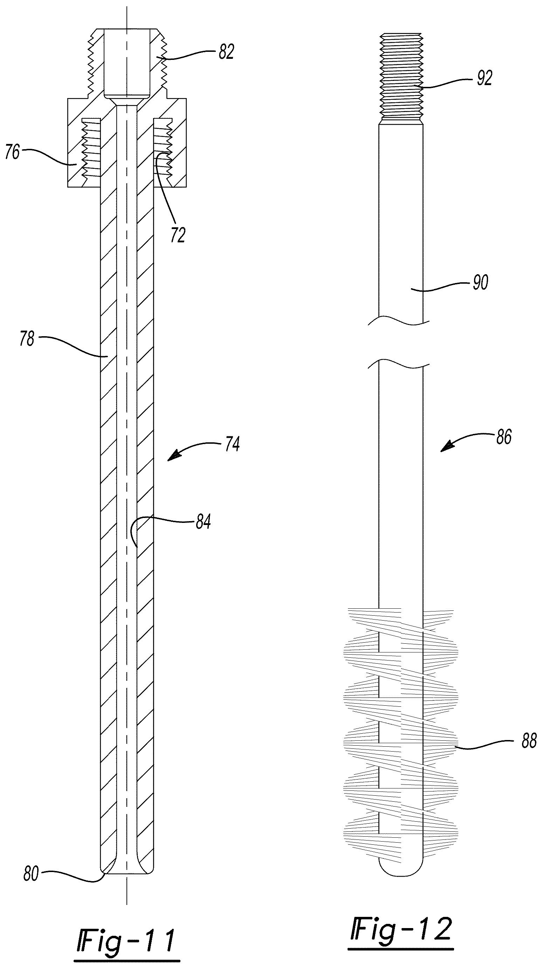

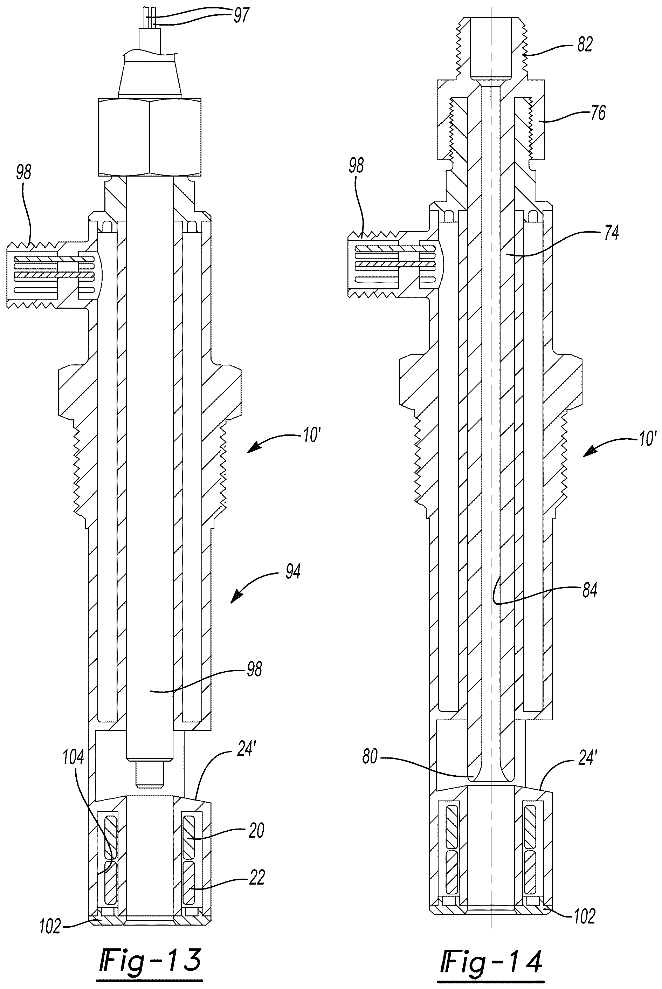

Various features described above can also be used in the embodiment 10' illustrated in Figs. 7 - 15. In this embodiment a resealable cap 70 is removably threaded to external threads 72 on the top of section 12'. As will appear, this construction allows access to the through hole 18' that allows manual cleaning of the sensing area of the conductivity sensor 10'. For example, as shown in Figs. 11 and 14, a flushing attachment device 74 includes its own cap 76 which is threaded onto the external threads 72. Device 74 as an elongated shaft 78 terminating in a diverging flushing nozzle 80. A water hose or the like having a female fitting can be connected to the reduced annular topmost male fitting 82. Pressurized water can then be forced through the longitudinally extending bore 84, with the flushing nozzle 80 spraying the walls of the through hole 18' adjacent the coils 20', 22' to clean that area, as best shown in Fig. 14.

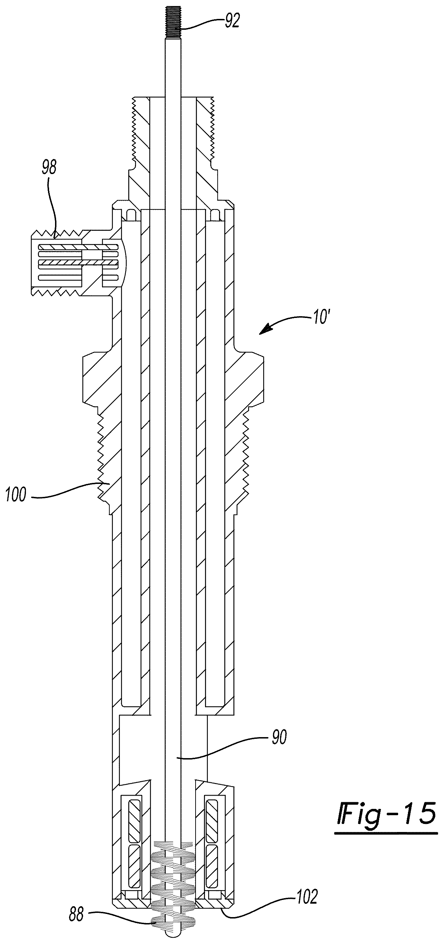

Another technique for cleaning the conductivity sensor 10' is shown in Figs. 12 and 15. Here, a cleaning brush 86 is provided with bristles 88 connected to a lower end of an elongated shaft 90 with a threaded upper threaded end 92. As shown in Fig. 15, a suitable tool serving as a handle can be threaded to end 92 and the brush bristles 88 can be inserted into the bore of the sensor body through the through hole 18'. Up-and-down reciprocating motion carries the bristles through the sensing area between the coils 20', 22' to manually clean that area.

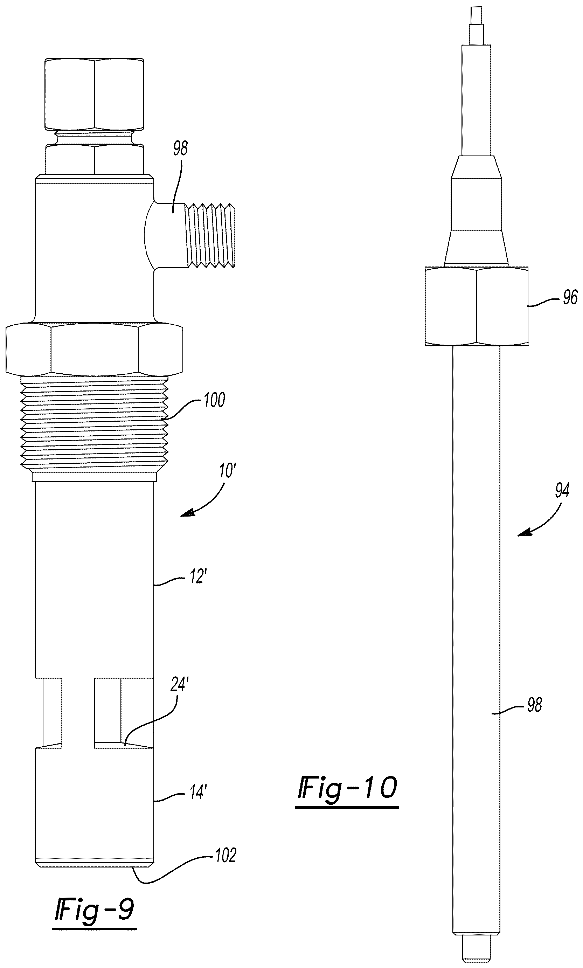

Turning now to Figs. 10 and 13, a secondary sensor 94 such as a borescope, temperature sensor, pH sensor, turbidity sensor or the like can be conveniently attached to the conductivity sensor 10'. The secondary sensor 94 similarly contains its own cap 96 which is threaded onto the end 82. The secondary sensor 94 includes an elongated shaft 98 terminating at its end which carries, for example, a fast response RTD temperature sensor or ultrasonic clog sensor. Alternatively, the construction of the secondary sensor 94 provides access so that a laser, ultrasonic or other sensor can be permanently or temporarily installed to check if the through hole 18 ' surrounding the coils is partially or fully blocked. Wires 97 from the sensing device are routed through the shaft 98 and protrude through the top of the secondary sensor 94.

As shown perhaps best in Figs. 9 and 13-15, an electrical connector 98 such as an M12 connector is provided on an upper side portion of section 12'. The wires 50 (a-c) can be conveniently routed through the pillars from the sensing coils to terminals in the connector 98. Connector 98 provides a convenient way to access information from the sensing coils when the sensor is mounted to a pipe or the like via the external threads 100 midway along the sensor section 12'.

A sonic welded cap 102 holds coils, 20, 22 in place in the other lower section 14. Thus, during assembly, the annular drive coil 20 can be slid into the annular channel 104, followed by the receiving coil 22. Then, the cap 102 is welded in place to hold the coils.

In operation, the conductivity sensor 10 is placed into the liquid to be measured. Conductivity sensor 10 is ideally suited for measuring resistivity/conductivity of liquids ranging from pure and ultrapure water to sea water, rinse water and chemical solutions, it is important that the conductivity sensor 10 be easily cleaned. In contrast to the design of some conventional conductivity sensors which typically requires removal of the sensor from the sensing environment for cleaning, the design of the sensor 10 maintains the cleanliness of the environment around the sensing coils 20, 22 during operation, as well as providing a construction that facilitates manual cleaning of the sensor 10.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A conductivity sensor comprising:
- an elongated cylindrical body having major axis with an upper section and a lower section spaced from the upper section, the body having a through hole in the lower section;
- a drive coil and a receiving coil in the lower section;
- a plurality of pillars connecting a top of the lower section with a bottom of the upper section of the body while leaving radially extending gaps therebetween to allow fluid to flow through the gaps and then pass adjacent to the drive coil and receiving coil and through the through hole in the lower section; and
- the pillars having holes therein providing conduits for wires extending from the drive coil and receiving coil to external measurement circuitry.

2. The conductivity sensor according to claim 1, wherein the pillars have radially inwardly extending fins configured to create turbulence in the fluid to be measured as the fluid passes the drive coil and receiving coil.

3. The conductivity sensor according to claim 2, wherein the fins are asymmetric with sides of different lengths.

4. The conductivity sensor according to claim 1, wherein the conductivity sensor further comprises:
- a U-shaped thermowell located in the top portion of the body above the gaps, the thermowell having sidewalls connected by a bridge member located adjacent to the gaps, the sidewalls being thermally isolated by spaces between the sidewalls and the top portion of the body; and
- a temperature sensor located on the bridge member.

5. The conductivity sensor according to claim 4, wherein the top section has a shelf internally of the body, with the thermowell having a flange resting on the shelf.

6. The conductivity sensor according to claim 5, wherein the conductivity sensor further comprises an O ring between the thermowell and the body of the top section, the O ring being located beneath the flange.

7. The conductivity sensor according to claim 1, wherein the conductivity sensor further comprises a downwardly extending conical flow diverter located above the gaps configured to generate turbulent flow of the fluid.

8. The conductivity sensor accoring to claim 7, wherein in the flow diverter extends from lower portions of a thermowell.

9. The conductivity sensor according to claim 1, wherein upper shoulders of the lower section extend radially inwardly and are angled upwardly configured to prevent debris from the liquid to spread towards the through hole when pipeline flow is turned off.

10. The conductivity sensor according to claim 1, wherein the conductivity sensor further comprises a removable resealable cap on top portions of the upper section providing selective access to the bore.

11. The conductivity sensor according to claim 10, wherein the conductivity sensor further comprises a flushing device removably extending through the bore, the flushing device having a nozzle at its end configured to clean the through hole adjacent the drive coil and receiving coil.

12. The conductivity sensor according to claim 10, wherein the conductivity sensor further comprises a cleaning brush removably extending through the bore, the cleaning brush having bristles at its end configured to clean the through hole adjacent the drive coil and receiving coil.

13. The conductivity sensor according to claim 1, wherein a top portion of the upper section includes a ribbon cable connector.

14. The conductivity sensor according to claim 13, wherein the top portion of the upper section includes a horizontal wire board on which the ribbon cable connector is mounted, with terminal pads radially outwardly extending from the ribbon cable connector.
